# EUROPEAN PATENT APPLICATION

(11) **EP 2 229 896 A1**
(43) Date of publication of application: **22.09.2010**
(21) Application number: 10250488.3
(22) Date of filing: 16.03.2010
(51) Int. Cl.: A61B 17/34

(54) **Access port including centering feature**

(30) Priority: 17.03.2009 US 160733 P; 04.03.2010 US 717268
(71) Applicant: Tyco Healthcare Group LP, North Haven, CT 06473 (US)
(72) Inventor: Abrams, Michael E., New York, NY 10282 (US)
(74) Representative: Soames, Candida Jane

(57) **Abstract**

An access port (100) includes a housing (110) defining a longitudinal axis (130) and having proximal and distal ends, and an interior wall defining a longitudinal opening (120) adapted for passage of a surgical object (200), an object seal (140) disposed in mechanical cooperation with the housing (110) and being configured to create a substantially fluid-tight seal around a surgical object (200) inserted through the object seal (140) and a centering mechanism (150,170,160) mounted to the housing (110). The centering mechanism includes at least one centering element (150 or 170) extending at least in a general longitudinal direction within the longitudinal opening (120) and a substantially annular ring (170) mounted to the at least one centering element. The at least one centering element (150 or 170) is positioned and dimensioned to engage the surgical object (200) during passage of the object through the longitudinal opening (120) and is capable of radial outward deflective movement relative to the longitudinal axis (130) in response to an outward force exerted by the surgical object (200) during eccentric manipulation of the surgical object (200). The annular ring (160) is adapted for radial movement during corresponding radial movement of the at least one centering element (150 or 170) upon eccentric manipulation of the surgical object (200), to thereby engage the interior wall and apply a generally inward force counteracting the outward force exerted by the surgical object (200) tending to bias the surgical object (200) toward a generally aligned position with respect to the longitudinal axis (130).

## Description

### CROSS REFERENCE TO RELATED APPLICATION

The present application claims the benefit of and priority to U.S. Provisional Application Serial No. 61/160,733 filed on March 17, 2009, the entire contents of which arc incorporated herein by reference.

### BACKGROUND

### Technical Field

The present disclosure relates to an access port which is adapted to allow the introduction of surgical instrumentation into a patient's body.

### Description of the Related Art

In laparoscopic procedures, surgery is performed in the interior of the abdomen through a small incision; in endoscopic procedures, surgery is performed in any hollow viscus of the body through a narrow tube or cannula inserted through a small entrance incision in the skin. Laparoscopic and endoscopic procedures generally require that any instrumentation inserted into the body be sealed, i.e. provisions must be made to ensure that gases do not enter or exit the body through the incision as, for example, in surgical procedures in which the surgical region is insufflated. Moreover, laparoscopic and endoscopic procedures often require the surgeon to act on organs, tissue, and vessels far removed from the incision, thereby requiring that any instruments used in such procedures be relatively long and narrow.

For such procedures, the introduction of a tube into certain anatomical cavities such as the abdominal cavity is usually accomplished by use of a trocar assembly made up of a cannula assembly and an obturator assembly. Since the cannula assembly provides a direct passage for surgical instrumentation from outside the patient's body to access internal organs and tissue, it is important that the cannula assembly maintain a relatively gas-tight interface between the abdominal cavity and the outside atmosphere. The cannula assembly generally includes a cannula attached to a cannula housing containing a seal assembly adapted to maintain a seal across the opening of the cannula housing.

Since surgical procedures in the abdominal cavity of the body require insufflating gases to raise the cavity wall away from vital organs, the procedure is usually initiated by use of a Verres needle through which a gas such as CO₂ is introduced into the body cavity, thereby creating a pneumoperitoneum. The gas provides a positive pressure which raises the inner body wall away from internal organs, thereby providing the surgeon with a region within which to operate and avoiding unnecessary contact with the organs by the instruments inserted through the cannula assembly. An obturator of the obturator assembly is inserted into the cannula assembly and used to puncture the abdominal wall. Following removal of the obturator assembly from the cannula assembly, laparoscopic or endoscopic surgical instruments may be inserted through the cannula assembly to perform surgery within the abdominal cavity.

Generally in the context of insufflatory surgical procedures, there are two sealing requirements for cannula assemblies. The first requirement is to provide a substantially fluid-tight seal when an instrument is not being introduced into or is not already present in the cannula. The second requirement is to provide a substantially fluid-tight seal when an instrument is being introduced into or is already present in the cannula. Additionally, as endoscopic and laparoscopic surgical procedures and techniques have advanced, it has become desirable to accommodate surgical instrumentation of varying outside diameters through a single cannula assembly in a given surgical procedure, thereby minimizing the number of cannula required and facilitating efficiency in the surgical procedure.

### SUMMARY

In accordance with a preferred embodiment, an access port includes a housing defining a longitudinal axis and having proximal and distal ends, and an interior wall defining a longitudinal opening adapted for passage of a surgical object, an object seal disposed in mechanical cooperation with the housing and being configured to create a substantially fluid-tight seal around a surgical object inserted through the object seal and a centering mechanism mounted to the housing. The centering mechanism includes at least one centering element extending at least in a general longitudinal direction within the longitudinal opening and a substantially annular ring mounted to the at least one centering element,. The at least one centering element is positioned and dimensioned to engage the surgical object during passage of the object through the longitudinal opening and is capable of radial outward deflective movement relative to the longitudinal axis in response to an outward force exerted by the surgical object during eccentric manipulation of the surgical object. The annular ring is adapted for radial movement during corresponding radial movement of the at least one centering element upon eccentric manipulation of the surgical object, to thereby engage the interior wall and apply a generally inward force counteracting the outward force exerted by the surgical object tending to bias the surgical object toward a generally aligned position with respect to the longitudinal axis.

In one embodiment, a plurality of centering elements is provided. An annular ring may be mounted to move within the longitudinal opening of the housing. The centering elements each may include a proximal end segment secured to the housing and a distal end segment secured to the annular ring. The annular ring may be adapted for radial movement and longitudinal movement with respect to the longitudinal axis. The centering elements may be coaxially arranged about the longitudinal axis. The centering elements may be each dimensioned to have an intermediate bow segment between the proximal and distal end segments. The bow segment may define a substantially curved configuration. The object seal may define a substantially conical segment with the object seal being at least partially disposed within the centering elements.

In another embodiment, a surgical cannula assembly includes a cannula housing, a cannula member extending from the cannula housing, and defining a longitudinal axis and having a longitudinal opening for reception and passage of a surgical object, a plurality of centering elements mounted to the cannula housing in coaxial arrangement with the longitudinal axis and an object seal at least partially disposed within an inner boundary defined within the centering elements and being adapted to create a substantially fluid-tight seal around the surgical object. The centering elements may be positioned and dimensioned to engage the surgical object during passage of the object through the longitudinal opening and capable of radial outward deflective movement relative to the longitudinal axis from an initial position to a radial outward position in response to an outward force exerted by the surgical object during eccentric manipulation of the surgical object. The centering elements may be normally biased toward the initial position to bias the surgical object toward a generally aligned position with respect to the longitudinal axis.

The object seal may define a generally tapered segment, e.g., a generally conical segment extending along the longitudinal axis. A substantially annular ring may be mounted to the centering elements. The annular ring may be adapted for radial movement during corresponding radial movement of the centering elements upon eccentric manipulation of the surgical object. The ring may be adapted to move in a radial and longitudinal direction relative to the longitudinal axis. The ring may be adapted to engage an interior wall of the cannula housing and apply a generally inward force counteracting an outward force exerted by the surgical object during eccentric movement of the surgical object, to thereby tend to bias the surgical object toward a generally aligned position with respect to the longitudinal axis.

### BRIEF DESCRIPTION OF THE DRAWINGS

Various embodiments of the present disclosure are described herein with reference to the drawings wherein:

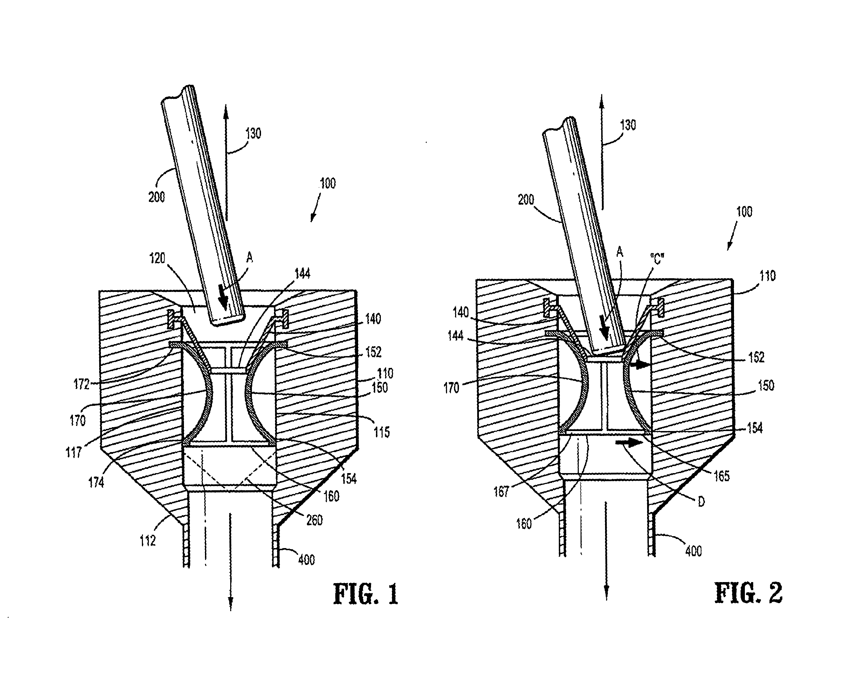

**Fig. 1** is a longitudinal cross-sectional view of the access port illustrating initial insertion of an instrument .

**Fig**. **2** is a view similar to the view of **Fig. 1** illustrating eccentric manipulation of the instrument within the access port and relative to the longitudinal axis of the access port;

**Fig. 3** is a view similar to the view of **Fig. 1** illustrating the instrument in substantial alignment with the longitudinal axis; and

**Fig. 4** is a longitudinal cross-sectional view of the access port illustrating a relatively large instrument introduced within the access port.

### DETAILED DESCRIPTION

The access port of the present disclosure, either alone or in combination with a cannula assembly, provides a substantially fluid-tight seal between a body cavity of a patient and the outside atmosphere. The access port of the present disclosure is configured to receive instruments of varying diameter. The centering mechanism includes ribs which assist in maintaining a substantially symmetrical position of a surgical instrument with respect to a longitudinal axis during insertion into the access port and cannula assembly. The ribs also help maintain the substantially symmetrical position of an instrument disposed within the access port while the instrument is disposed therethrough.

The access port of the present disclosure contemplates the introduction of various types of instrumentation adapted for insertion through a trocar and/or cannula assembly while maintaining a substantially fluid-tight interface about the instrument to help preserve the atmospheric integrity of a surgical procedure from gas and/or fluid leakage. Specifically, the access port includes at least two ribs which bias an instrument entering the port such that the instrument enters the port or, is normally biased toward a substantially symmetrical relation with the longitudinal axis. This feature of the present disclosure minimizes the entry and exit of gases and/or fluids to/from the body cavity. Examples of instrumentation include, but are not limited to, clip appliers, graspers, dissectors, retractors, staplers, laser probes, photographic devices, endoscopes and laparoscopes, tubes, and the like. Such instruments will collectively be referred to as "instruments" or "instrumentation."

In the following description, as is traditional, the term "proximal" refers to the portion of the device closer to the operator while the term "distal" refers to the portion of the device farther from the operator.

Referring now to the drawings, **Fig. 1** shows access port **100** including a housing **110** defining a longitudinal axis 1**30.** Housing **110** includes an orifice seal **140** having an aperture **144** therein. Orifice seal **140** may be made from a low durometer elastomer and/or include a hydrophilic coating. Orifice seal **140** may be a conical or tapered seal extending along the longitudinal axis **130**. Opposing ribs **150** and **170** are rigidly attached to respective inner surfaces **115**, **117** of housing **110** adjacent their respective proximal ends **152, 172** and attached to a ring **160** adjacent their respective distal ends **154**, **174**. Ring **160** is free of engagement with housing **110** and is thus free to "move" within the housing in both a radial and a longitudinal direction. Ribs **150**, **170** and ring **160** form a centering mechanism tending to bias the surgical object into general alignment with the longitudinal axis **130**. Ribs **150, 170** may be formed of any material having sufficient resiliency to permit deflection and return to its initial position. Ribs **150**,**170** may be secured to an internal surface of housing **110** by conventional means including adhesive, cements, pins, fasteners or the like. Ring **160** may be secured to ribs **150**, **170** in a similar manner. Suitable materials may include polymeric material, spring steel, titanium or the like. Ring 160 may be formed of a more rigid material. Ring **160** may define a diameter substantially approximating the internal dimension of inner wall **117**.

A distal end **112** of housing **110** is shown monolithically formed with a cannula assembly **400**. Access port **100** may also be configured to mechanically engage cannula assembly **400** in a variety of ways including, but not limited to, through a bayonet lock or threaded connection. Access port **100** and/or cannula assembly 400 may include a second seal **260** (shown in phantom) which provides a substantially fluid-tight seal in the absence of a surgical instrument passing therethrough.

While two ribs **150** and **170** are shown, it is envisioned and within the scope of the present disclosure that access port **100** includes more (e.g., four ribs at 90 degree radial intervals) or fewer than two ribs.

Opposing ribs **150**, **170** may be bow-like in its normal state such that the gap distance between opposing ribs **150** and **170** at its narrowest point is slightly less than the smallest diameter instrument which is likely to be inserted into housing **110**. For example, if the minimum diameter instrument which is likely to be introduced into housing **110** is about 5 mm, the gap distance between opposing ribs **150** and **170**, at its narrowest point, could be about 4.5mm in an at-rest position. Opposing ribs **150**, **170** may also be configured such that in a flexed position, opposing ribs **150** and **170** can accommodate instruments having a diameter up to about 12mm. Thus, access port **100** may be adapted to receive surgical instrumentation having a diameter in the range of about 5mm to about 12mm. The capability of access port being adapted to accommodate smaller and larger diameter instruments is also envisioned.

Opposing ribs **150**, **170** and ring **160** cooperate to assist in maintaining a substantially symmetrical relation of a surgical instrument **200** with respect to a longitudinal axis 130., thus, minimizing of leakage of fluids between orifice seal **140** and the object, e.g., once disposed through housing **110**, surgical instrument **200** is radially held in place, substantially symmetrical about longitudinal axis **130** via opposing ribs **150**,**170** and ring **160.** The operation of ribs **150**, **170** and ring **160** before, during and after insertion of a surgical instrument into housing **110** will be described more fully below.

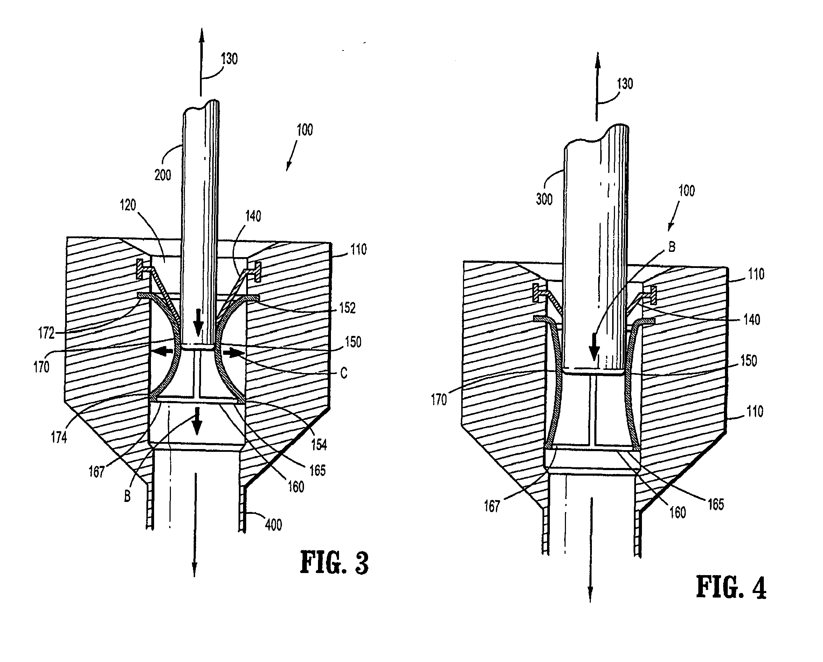

The use of access port **100** will now be described in detail with reference to Figs. 1-4. Fig. 1 shows instrument **200** being introduced into a channel **120** defined within housing **110** of access port **100** in an asymmetrical direction about longitudinal axis **130** (arrow A). Asymmetrical may be interpreted as at least including offset, angulated, lateral or the like with respect to the longitudinal axis **130**. Before insertion of instrument **200,** ribs **150**, **170** and ring **160** are in an initial at-rest position, as shown in Fig. 1. As instrument **200** is further advanced distally in the direction of arrow A, instrument **200** contacts rib **150**. Upon contacting rib **150**, as shown in Fig. 2, instrument **200** exerts both radial and longitudinal forces force on rib **150**. Since rib **150** is rigidly attached to inner surface **115** of housing **110** adjacent proximal end **152**, the force applied to rib **150** causes rib **150** to bow or deflect in the direction of arrow C which initially also causes translation of ring **160** in an axial direction, and imparts radial movement of the ring **160** relative to the longitudinal axis **130** and toward interior wall **115**. Upon radial movement of ring **160** a predetermined distance "d", ring **160** is forced into interior wall **115** in secured relation therewith through, e.g., a frictional relationship created between ring **160** and inner wall **115**. With ring **160** secured relative to interior wall **115**, the ring **160** may no longer translate in the axial direction. As a result, a counterforce is created within rib **150**, biasing the rib **150** and the surgical object toward an aligned position with respect to the longitudinal axis. Once the surgical object is aligned, the surgical object may be advanced through aperture **144** of orifice seal **140** creating a fluid-tight relationship around instrument **200**, as depicted in Figure 3. If alignment is maintained, the surgical object may be advanced with ribs **150**, **170** deflecting to cause corresponding axial movement of ring **160**. For example, when an even load is applied to ribs **115, 117** coaxial arrangement of ring **160** with respect to longitudinal axial **130** thus permitting the ring **160** and instrument **200** to translate in an axial direction. However, if during any time, the surgical object is laterally manipulated or angulated relative to the longitudinal axis, ribs **150**, **170** will deflect causing corresponding radial movement of ring **160** into engagement with inner wall **115**. In this position, advancing movement of the surgical object is substantially prevented until the surgical object is in general alignment with the longitudinal axis **130** and, e.g., equal forces are applied to ribs **150**, **170**.

Ring **160** may include a textured outer surface, e.g., such as ribs, protrusions, teeth or the like to facilitate engagement with the interior wall **115** of inner wall **115.** Ring 160 may also have an elastomeric outer surface to facilitate frictional engagement with interior wall **115.** Interior wall **115** may include similar surfaces.

Fig. 4 illustrates an instrument **300** having a relatively large diameter (e.g., about 12mm). Instrument **300** causes ribs **150** and **170** to bow radially outward, causing ring **160** to be displaced distally as instrument **300** is inserted into housing **110** in a substantially symmetrical manner with respect to longitudinal axis **130**. Although the radial displacement of ribs **150** and **170** and the distal displacement of ring **160** would be greater than in the case of instrument **200**, ribs **150** and **170** and ring **160** would operate in substantially the same manner. Instrument **300** would be held in place substantially symmetrical about longitudinal axis **130** and ribs **150**, **170** and ring **160** would assist in minimizing asymmetrical insertion of instrument **300** and would also assist in minimizing movement of instrument **300** away from longitudinal axis **130.**

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. An access port comprising:
a housing defining a longitudinal axis and having proximal and distal ends, the housing having an interior wall defining a longitudinal opening adapted for passage of a surgical object;
an object seal disposed in mechanical cooperation with the housing, the object seal configured to create a substantially fluid-tight seal around a surgical object inserted through the object seal; and
a centering mechanism mounted to the housing, the centering mechanism including:
at least one centering element extending at least in a general longitudinal direction within the longitudinal opening, the at least one centering element positioned and dimensioned to engage the surgical object during passage of the object through the longitudinal opening and capable of radial outward deflective movement relative to the longitudinal axis in response to an outward force exerted by the surgical object during eccentric manipulation of the surgical object; and
a substantially annular ring mounted to the at least one centering element, the annular ring adapted for radial movement during corresponding radial movement of the at least one centering element upon eccentric manipulation of the surgical object, to thereby engage the interior wall and apply a generally inward force counteracting the outward force exerted by the surgical object tending to bias the surgical object toward a generally aligned position with respect to the longitudinal axis.

2. The access port of claim 1 including a plurality of centering elements.

3. The access port of claim 1 or claim 2 wherein the annular ring is mounted to move within the longitudinal opening of the housing.

4. The access port of any preceding claim wherein the centering elements each include a proximal end segment secured to the housing and a distal end segment secured to the annular ring.

5. The access port of claim 4 wherein the annular ring is adapted for radial movement and longitudinal movement with respect to the longitudinal axis.

6. The access port of claim any preceding claim wherein the centering elements are coaxially arranged about the longitudinal axis.

7. The access port according to any preceding claim wherein the centering elements are each dimensioned to have an intermediate bow segment between the proximal and distal end segments, the bow segment defining a substantially curved configuration.

8. The access port of any preceding claim wherein the object seal defines a substantially conical segment, the object seal at least partially disposed within the centering elements.

9. A surgical cannula assembly, which comprises:
a cannula housing;
a cannula member extending from the cannula housing, the cannula housing and the cannula member defining a longitudinal axis and having a longitudinal opening for reception and passage of a surgical object;
a plurality of centering elements mounted to the cannula housing in coaxial arrangement with the longitudinal axis, the centering elements positioned and dimensioned to engage the surgical object during passage of the object through the longitudinal opening and capable of radial outward deflective movement relative to the longitudinal axis from an initial position to a radial outward position in response to an outward force exerted by the surgical object during eccentric manipulation of the surgical object, the centering elements normally biased toward the initial position to bias the surgical object toward a generally aligned position with respect to the longitudinal axis; and
an object seal at least partially disposed within an inner boundary defined within the centering elements, the object seal configured to create a substantially fluid-tight seal around the surgical object.

10. The surgical cannula assembly of claim **9** wherein the object seal defines a generally tapered segment extending along the longitudinal axis.

11. The surgical cannula assembly of claim 10 wherein the object seal defines a generally conical segment.

12. The surgical cannula assembly of any of claims 9 to 11 including a substantially annular ring mounted to the centering elements, the annular ring adapted for radial movement during corresponding radial movement of the centering elements upon eccentric manipulation of the surgical object.

13. The surgical cannula assembly according to claim 12 wherein the ring is adapted to move in a radial and longitudinal direction relative to the longitudinal axis.

14. The surgical cannula assembly according to claim 13 wherein the ring is adapted to engage an interior wall of the cannula housing and apply a generally inward force counteracting an outward force exerted by the surgical object during eccentric movement of the surgical object, to thereby tend to bias the surgical object toward a generally aligned position with respect to the longitudinal axis.
